# EUROPÄISCHE PATENTANMELDUNG

(11) **EP 3 878 832 A1**
(43) Veröffentlichungstag der Anmeldung: **15.09.2021**
(21) Anmeldenummer: 20162338.6
(22) Anmeldetag: 11.03.2020
(51) Int. Cl.: C07C 29/88, C07C 29/94, C07C 31/20, C07C 67/05, C07C 69/675, C07C 69/16, C07C 69/08

(54) **VERFAHREN ZUR DESODORISIERUNG VON 1,2-ALKANDIOLEN DURCH DESTILLATION**

(71) Anmelder: Evonik Operations GmbH, 45128 Essen (DE)
(72) Erfinder: WERNER, Bettina, Eiken (CH); RINKER, Stefanie, 46284 Dorsten (DE); GÄRTNER, Felix, 45721 Haltern am See (DE)
(74) Vertreter: Evonik Patent Association

(57) **Zusammenfassung**

Die vorliegende Erfindung betrifft ein Verfahren zur Desodorierung von 1,2-Alkandiolen. Das Verfahren zeichnet sich dadurch aus, dass übelriechende organische Verunreinigungen in Mischungen umfassend 1,2-Alkandiole, wie sie üblicherweise nach der Synthese aus den entsprechenden 1-Alkenen erhalten werden, gereinigt werden. Die Desodorierung der 1,2-Alkandiole erfolgt dabei durch ihre destillative Abtrennung von den organischen Verunreinigungen, wobei die zu reinigende Mischung vor oder während der destillativen Abtrennung mit einem Zusatz vermischt wird. Der Zusatz ist mindestens eine polymere Sulfonsäure. Die nach dem erfindungsgemäßen Verfahren desodorierten 1,2-Alkandiole eignen sich besonders gut zum Einsatz in kosmetischen Produkten oder in Reinigungsprodukten für den Haushalt.

## Beschreibung

Die vorliegende Erfindung betrifft ein Verfahren zur Desodorierung von 1,2-Alkandiolen. Das Verfahren zeichnet sich dadurch aus, dass übelriechende organische Verunreinigungen in Mischungen umfassend 1,2-Alkandiole, wie sie üblicherweise nach der Synthese aus den entsprechenden 1-Alkenen erhalten werden, gereinigt werden. Die Desodorierung der 1,2-Alkandiole erfolgt dabei durch ihre destillative Abtrennung von den organischen Verunreinigungen, wobei die zu reinigende Mischung vor oder während der destillativen Abtrennung mit einem Zusatz vermischt wird. Der Zusatz ist mindestens eine polymere Sulfonsäure. Die nach dem erfindungsgemäßen Verfahren desodorierten 1,2-Alkandiole eignen sich besonders gut zum Einsatz in kosmetischen Produkten oder in Reinigungsprodukten für den Haushalt.

### 1. Hintergrund der Erfindung

1,2-Alkandiole finden vielfache Anwendung in der Kosmetik, in Haushaltsprodukten und in der Technik. Sie weisen befeuchtende Eigenschaften auf, können als Lösungsvermittler eingesetzt werden und sind chemisch stabil. Diese und andere Eigenschaften prädestinieren sie für die genannten Anwendungen.

EP 0 655 904 B1 beschreibt den Einsatz von 1,2-Alkandiolen, insbesondere 1,2-Pentandiol, in hautbefeuchtenden Mitteln.

EP 1 478 231 B1 beschreibt Mischungen aus mindestens zwei 1,2-Alkandiolen, beispielsweise 1,2-Hexandiol und 1,2-Octandiol, mit verbesserten antimikrobiellen Eigenschaften.

EP 1 598 064 B1 beschreibt den Einsatz von Mischungen aus einem Alkandiol und einer hydroxylierten Säure gegen Akne.

EP 2 842 607 A1 beschreibt die Verwendung von Mischungen aus Alkandiolen, Resorcinolderivaten und Bisabolol in hautaufhellenden Produkten.

WO 2006/069953 A1 beschreibt Mischungen aus mindestens zwei Komponenten, die aus 1,2-Pentandiol, 1,2-Hexandiol und 1,2-Octandiol ausgewählt sind. Solche Mischungen können als Formulierungen zur Hautbefeuchtung verwendet werden.

WO 2019/029808 A1 beschreibt ein Verfahren zur Herstellung besonders reiner 1,2-Alkandiole, die sich aufgrund ihrer hohen Reinheit gut zum Einsatz in kosmetischen Formulierungen und Haushaltsprodukten eignen.

DE 32 29 084 A1 beschreibt ein Verfahren zur Herstellung vicinaler Diole, in welchem 1-Alkene mit Wasserstoffperoxid und Ameisensäure umgesetzt und die so erhaltenen Formiatester dann durch Decarbonylierung in die entsprechenden 1,2-Alkandiole überführt werden. Die Reinigung der 1,2-Alkandiole erfolgt durch Destillation.

KR-10-2015-0122854 A beschreibt ein Verfahren zur Herstellung von 1,2-Hexandiolen mit hoher Reinheit. In dem Verfahren wird 1-Hexen mit Ameisensäure und H₂O₂ in Gegenwart von Eisen(III)-Salzen wie FeCl₃ umgesetzt.

EP 1 671 936 A2 beschreibt ein Verfahren zur Herstellung von vicinalen Alkandiolen und Alkantriolen, in dem Monoolefine mit H₂O₂ und Ameisensäure umgesetzt werden. Die erhaltenen Formiate werden in Gegenwart einer Arylsulfonsäure mit Methanol umgeestert und aus der entsprechenden Mischung das 1,2-Alkandiol destilliert.

Ein Problem bei der Synthese von 1,2-Alkandiolen über die beschriebenen Routen ist die Bildung von organischen Nebenprodukten. Dies ist problematisch, da diese 1,2-Alkandiolen typischerweise in Produkten für Endverbraucher verarbeitet werden. Schon allein deshalb besteht bei der Verarbeitung von 1,2-Alkandiolen in Verbrauchsgütern die Notwendigkeit, diese möglichst rein einzusetzen. Zusätzliche Brisanz erhält das Problem dadurch, dass ein Hauptanwendungsgebiet der 1,2-Alkandiole kosmetische Produkte sind. Hier ist die Abtrennung organischer Verunreinigungen, gerade wenn diese übelriechend sind, besonders wichtig.

Diese Problematik wird schon in der WO 2019/029808 A1 diskutiert, in denen als mögliche übelriechende organische Verunreinigungen Lactone, die sich während der Synthese der 1,2-Alkandiole aus 1-Alkenen bilden, identifziert werden.

Es wurde nun gefunden, dass sich eine andere Gruppe übelriechender organischer Verunreinigungen in einigen organischen Synthesen aus einer weiteren Quelle ergeben.

DE 199 29 196 A1 beschreibt ein Verfahren zur Herstellung von vicinalen Diolen aus den entsprechenden Alkenen mit H₂O₂ und Ameisen- und/oder Essigsäure zu den entsprechenden Estern. Die Umsetzung wird ohne Zusatz eines organischen Lösungsmittels durchgeführt, wodurch sich ein zweiphasiges Produktgemisch bildet. Diesem wird ein organisches Lösungsmittel zugegeben und überschüssiges Wasser und Ameisen- und Essigsäure azeotrop abdestilliert. Der Rückstand wird verseift und aus dem Rückstand wird Wasser mit einem organischen Lösungsmittel azeotrop abdestilliert.

Wie aus der DE 199 29 196 A1 ersichtlich, besteht bei der Umsetzung von vicinalen Diolen mit H₂O₂ und Ameisen- und/oder Essigsäure zu den entsprechenden Estern das Problem, dass sich dabei zwei Produktphasen bilden. Die organische Phase umfasst die intermediär gebildeten Mono- und Diester des Diols mit Ameisensäure bzw. Essigsäure. Die wässrige Phase umfasst überschüssiges H₂O₂ sowie nicht reagierte Essigsäure bzw. Ameisensäure.

Diese Ausbildung mehrerer Phasen verkompliziert die Aufarbeitung der erhaltenen Reaktionsmischung, ein Problem, das in der DE 199 29 196 A1 adressiert wird. Daneben erschwert sie aber auch die Umsetzung der Edukte zum Produkt. Zu diesem Zweck werden der Reaktionsmischung Phasentransferkatalysatoren zugesetzt, welche die Umsetzung beschleunigen.

X. Wu & B. Li, Chinese Chemical Letters 2014, 25, 459-462 beschreiben den Zusatz von quartären Alkylammoniumsalzen zur Umsetzung vicinaler Alkene mit H₂O₂ und Ameisensäure.

G. Lewandowski, Polish Journal of Chemical Technology 2013, 15, 96-99 (im Folgenden "Lewandowski") beschreibt ebenfalls quartärer Ammoniumsalze bei der Herstellung vicinaler Diole aus 1,5,9-Cyclododecatrien mit H₂O₂ und Dodecawolframphosphorsäure.

Der Einsatz von Phasentransferkatalysatoren in der Synthese von vicinalen Diolen stellt eine Verbesserung des Verfahrens dar, da sie zu einer erhöhten Reaktionsgeschwindigkeit führt. Sie führt allerdings zu einem weiteren Problem, was gerade dann berücksichtigt werden muss, wenn die erhaltenen vicinalen Alkandiole in Kosmetika und Haushaltsmitteln eingesetzt werden. So wurde auch bei den unter Einsatz von Phasentransferkatalysatoren synthetisierten vicinalen Alkandiolen ein schlechter Geruch beobachtet, der auch nach erneuter Destillation nicht ganz entfernt werden kann. Dieser Geruch taucht teilweise auch im erhaltenen 1,2-Alkandiol auf.

Die Erfinder der vorliegenden Erfindung vermuteten, dass dieser Geruch auf den Einsatz der quarternären Ammoniumsalze zurückzuführen ist, die sich während der Destillation des Produkts zersetzen. Es wurde weiterhin vermutet, dass bei der destillativen Reinigung aminische Zersetzungsprodukte mitgeschleppt werden, und sofort oder nach einer Weile zu dem schlechten Geruch im 1,2-Alkandiol führen.

Die Aufgabe der vorliegenden Erfindung bestand demnach darin, ein verbessertes Verfahren zur Herstellung von 1,2-Alkandiolen zur Verfügung zu stellen, welches den genannten Nachteil nicht aufweist und insbesondere, bei Beibehaltung der Vorteile, die eine Phasentransferkatalyse der Reaktion mit sich bringt, zu 1,2-Alkandiolen mit verbesserter geruchlicher Qualität führt.

Es wurde nun überraschend ein Verfahren gefunden, was die vorgenannte Aufgabe löst.

### 2. Detaillierte Beschreibung der Erfindung

Das erfindungsgemäße Verfahren ist eines zur Desodorierung mindestens eines Alkandiols der Formel **(I)** mit 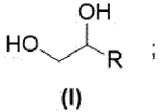 wobei R ein unverzweigter oder verzweigter, bevorzugt unverzweigter, Alkylrest mit 1 bis 10, insbesondere 3, Kohlenstoffatomen ist,
wobei das mindestens eine Alkandiol der Formel **(I)** in einer Mischung **M** umfassend mindestens ein Alkandiol der Formel **(I)** und organische Verunreinigungen **V** bereitgestellt wird, und gemäß dem Schritt (α), (β) oder (γ) mindestens teilweise von den organische Verunreinigungen **V** abgetrennt wird:
(α) (α1) Vermischen der Mischung **M** mit mindestens einem Zusatz **Z,** wodurch eine Mischung **Mₐ₁** umfassend mindestens ein Alkandiol der Formel **(I)**, mindestens einen Zusatz **Z** und die organischen Verunreinigungen **V** erhalten wird,
(α2) und mindestens teilweise destillative Abtrennung des mindestens einen Alkandiols der Formel **(I)** aus der Mischung **Mₐ₁;**
(β) (β1) Vermischen der Mischung **M** mit mindestens einem Zusatz **Z,** wodurch eine Mischung **Mₐ₁** umfassend mindestens ein Alkandiol der Formel **(I)**, mindestens einen Zusatz **Z** und die organischen Verunreinigungen **V** erhalten wird,
(β2) mindestens teilweise Abtrennung des Zusatzes **Z** aus der Mischung **Mₐ₁,** wodurch eine Mischung **Mₐ₂** umfassend mindestens ein Alkandiol der Formel **(I)**, mindestens einen Zusatz **Z** und die organischen Verunreinigungen **V** erhalten wird, wobei der Gehalt des Zusatzes **Z** in der Mischung **Mₐ₂** gegenüber **Mₐ₁** verringert ist,
(β3) und mindestens teilweise destillative Abtrennung des mindestens einen Alkandiols der Formel **(I)** aus der Mischung **Mₐ₂;**
(γ) mindestens teilweise destillative Abtrennung des mindestens einen Alkandiols der Formel **(I)** aus der Mischung **M,** wobei während der destillative Abtrennung des Alkandiols der Formel **(I)** aus der Mischung **M** mindestens ein Zusatz **Z** zur Mischung **M** zugegeben wird;
dadurch gekennzeichnet, dass der Zusatz **Z** mindestens eine polymere Sulfonsäure umfasst.

### 2.1 Destillative Reinigung des Alkandiols von organischen Verunreinigungen

Der kennzeichnende Schritt des erfindungsgemäßen Verfahrens umfasst die Bereitstellung des Alkandiols der Formel **(I)** in einer Mischung **M** umfassend neben dem Alkandiol der Formel **(I)** organische Verunreinigungen **V.**

Diese Mischung **M** kann auf verschiedene Möglichkeiten anfallen. Insbesondere wird sie jedoch durch eines der im Stand der Technik beschriebenen Verfahren erhalten, in denen das Alkandiol der Formel **(I)** ausgehend vom 1-Alken der nachfolgend genannten Formel **(II)** durch Umsetzung mit H₂O₂ und Ameisensäure oder Essigsäure umgesetzt, die erhaltenen Formiate bzw. Acetate verseift werden und das Rohprodukt destilliert wird. Das Destillat ist dann die Mischung **M.**

Ganz besonders effizient und deshalb bevorzugt ist das erfindungsgemäße Verfahren, wenn während dieser Synthese quartäre Alkylammoniumverbindungen eingesetzt werden, die sich dann insbesondere im Rohprodukt wiederfinden. Diese zersetzen sich mindestens teilweise während der Destillation und bilden aminische organische Verunreinigungen, die sich besonders gut mit dem erfindungsgemäßen Verfahren entfernen lassen. Daneben ist das erfindungsgemäße Verfahren aber auch geeignet, die in der WO 2019/029808 A1 beschriebenen übelriechenden Lactone zu entfernen.

Das erfindungsgemäße Verfahren ist aber auch zur Desodorierung von 1,2-Alkandiolen anwendbar, die in Mischungen vorliegen, die über andere Verfahren erhalten wurden, beispielsweise durch Epoxidierung eines Alkens mittels Wolframkatalyse und anschließender Ringöffnung zum 1,2-Alkandiol. Eine weitere Synthese ist auch durch eine Umsetzung des Alkens mit Natriumhypochlorit und saurer Öffnung des entstehenden Epoxids möglich.

Die organischen Verunreinigungen **V** lassen sich an ihrem von der menschlichen Nase als schlecht empfundenen Geruch erkennen, sind also übelriechend. Bei den organischen Verunreinigungen **V** handelt es sich demnach insbesondere um solche aus der Gruppe der Ketone, Lactone und Amine, ganz besonders um Amine.

Lactonische organischen Verunreinigungen **V** sind typischerweise organische ringförmige Verbindungen, die eine Estergruppe im Ring aufweisen, und die sich insbesondere bei der Reaktion des eingesetzten 1-Alkens mit H₂O₂ und der jeweiligen Säure zum Mono- oder Diester als Nebenprodukte ergeben oder bei der Zersetzung dieser Ester unter den Reaktionsbedingungen des erfindungsgemäßen Verfahrens. Bevorzugt handelt es sich bei lactonischen Verunreinigungen um Lactone mit 4 bis 14 Kohlenstoffatomen, insbesondere mit 4 bis 12 Kohlenstoffatomen. Organischen Verunreinigungen **V**, die Ketone sind, entstehen insbesondere bei der Reaktion des eingesetzten 1-Alkens mit H₂O₂ und der jeweiligen Säure zum Mono- oder Diester als Nebenprodukte oder bei der Zersetzung dieser Ester unter den Reaktionsbedingungen des erfindungsgemäßen Verfahrens. Bevorzugt weisen solche Ketone 4 bis 14 Kohlenstoffatomen, insbesondere 4 bis 12 Kohlenstoffatome auf. Ketone weisen einen Carbonylkohlenstoff auf, welcher an zwei Kohlenstoffatome gebunden ist.

Als aminische organische Verunreinigungen **V** kommen insbesondere solche in Frage, die sich bei der Zersetzung der erfindungsgemäßen quartären Ammoniumsalze ergeben. Dies können beispielsweise aliphatische Amine oder aromatische Amine sein, bevorzugt aliphatische Amine, wobei aliphatische Amine bevorzugt sekundäre oder tertiäre Alkylamine umfassen.

Im erfindungsgemäßen Verfahren wird das mindestens eine Alkandiol der Formel **(I)** von der in der Mischung **M** vorliegenden organische Verunreinigungen **V** mindestens teilweise abgetrennt. Das bedeutet, dass nach Durchführung des erfindungsgemäßen Verfahrens eine Zusammensetzung erhalten wird, in der der Massenanteil der organischen Verunreinigungen **V**, bezogen auf die Masse aller Alkandiole der Formel **(I)**, geringer ist als der Massenanteil der organischen Verunreinigungen **V**, bezogen auf die Masse aller Alkandiole der Formel **(I)**, in der Mischung **M.**

Insbesondere bedeutet dies, dass nach Durchführung des erfindungsgemäßen Verfahrens eine Zusammensetzung erhalten wird, in der der Massenanteil der organischen Verunreinigungen **V**, bezogen auf die Masse aller Alkandiole der Formel **(I)**, weniger als 50 %, bevorzugt weniger als 75 %, bevorzugter weniger als 90 %, bevorzugter weniger als 99 % des Massenanteils der organischen Verunreinigungen **V**, bezogen auf die Masse aller Alkandiole der Formel **(I)** in der Mischung **M,** beträgt.

Das bereitgestellte Alkandiol der Formel **(I)** in einer Mischung **M** umfassend mindestens ein Alkandiol der Formel **(I)** und organische Verunreinigungen **V** wird im erfindungsgemäßen Verfahren nach einem der folgenden Schritte (α), (β) oder (γ) mindestens teilweise von den organische Verunreinigungen **V** abgetrennt. Bevorzugt wird es nach einem der folgenden Schritte (α) oder (β), bevorzugter nach dem Schritt (β), mindestens teilweise von den organische Verunreinigungen **V** abgetrennt.

### 2.1.1 Schritt (α)

Als erste Möglichkeit wird die Mischung **M** in einem ersten Unterschritt (α1) mit mindestens einem Zusatz **Z** vermischt, wodurch eine Mischung **Mₐ₁** umfassend mindestens ein Alkandiol der Formel **(I)**, mindestens einen Zusatz **Z** und die organischen Verunreinigungen **V** erhalten wird.

Der Zusatz **Z** wird im Unterschritt (α1) dabei insbesondere in einer solchen Menge zugegeben, dass der Massenanteil aller Zusätze **Z** bezogen auf die Masse aller von der Mischung **M** umfassten Alkandiole **(I)** im Bereich 0.01 bis 10 Gew.-%, bevorzugt 0.1 bis 5 Gew.-%, bevorzugter 0.2 bis 3 Gew.-%, am bevorzugtesten 0.5 bis 2 Gew.-%, am allerbevorzugtesten 1 bis 1.5 Gew.-% liegt.

Im Anschluss an Unterschritt (α1) wird dann eine Mischung **Mₐ₁** umfassend mindestens ein Alkandiol der Formel **(I)**, mindestens einen Zusatz **Z** und die organischen Verunreinigungen **V** erhalten.

Aus der Mischung **Mₐ₁** wird dann im Unterschritt (α2) das mindestens eine Alkandiol der Formel **(I)** mindestens teilweise destillativ abgetrennt.

Die destillative Abtrennung des Alkandiols der Formel **(I)** kann nach dem Fachmann bekannten Verfahren erfolgen, wie zum Beispiel in der WO 2019/029808 A1, Absatz [0270] beschrieben.

Im Anschluss an die destillative Abtrennung wird dann die Zusammensetzung umfassend das gereinigte mindestens eine Alkandiol der Formel **(I)** erhalten.

### 2.1.2 Schritt (β)

Als zweite Möglichkeit wird die Mischung **M** in einem ersten Unterschritt (β1) mit mindestens einem Zusatz **Z** vermischt, wodurch eine Mischung **Mₐ₁** umfassend mindestens ein Alkandiol der Formel **(I)**, mindestens einen Zusatz **Z** und die organischen Verunreinigungen **V** erhalten wird.

Der Zusatz **Z** wird im Unterschritt (β1) dabei insbesondere in einer solchen Menge zugegeben, dass der Massenanteil aller Zusätze **Z** bezogen auf die Masse aller von der Mischung **M** umfassten Alkandiole **(I)** im Bereich 0.01 bis 10 Gew.-%, bevorzugt 0.1 bis 5 Gew.-%, bevorzugter 0.2 bis 3 Gew.-%, am bevorzugtesten 0.5 bis 2 Gew.-%, am allerbevorzugtesten 1 bis 1.5 Gew.-% liegt.

Im Anschluss an Unterschritt (β1) wird dann eine Mischung **Mₐ₁** umfassend mindestens ein Alkandiol der Formel **(I)**, mindestens einen Zusatz **Z** und die organischen Verunreinigungen **V** erhalten.

In einem Unterschritt (β2) wird dann der Zusatz **Z** aus der Mischung **Mₐ₁** wieder entfernt. Dies erfolgt insbesondere durch Dekantierung oder Filtration, insbesondere durch Filtration. In einer noch bevorzugteren Ausführungsform des Schrittes (β) wird zwischen den Unterschritten (β1) und (β2) die Mischung **Mₐ₁** gerührt, bevorzugt für mindestens 10 Sekunden bis 1 Stunde, bevorzugter 1 Minute bis 30 Minuten, noch bevorzugter 5 Minuten bis 10 Minuten. Dies stellt sicher, dass von der Mischung **Mₐ₁** umfasste Verunreinigungen **V** mit dem Zusatz **Z** reagieren und/oder adsorbiert werden.

Nach Durchführung des Unterschrittes (β2) wird eine Mischung **Mₐ₂** umfassend mindestens ein Alkandiol der Formel **(I)**, mindestens einen Zusatz **Z** und die organischen Verunreinigungen **V** erhalten, wobei der Gehalt des Zusatzes **Z** in der Mischung **Mₐ₂** gegenüber **Mₐ₁** verringert ist. Bevorzugt ist auch der Gehalt der organischen Verunreinigungen **V** in der Mischung **Mₐ₂,** bezogen auf den Gehalt aller Alkandiole der Formel **(I)** in der Mischung **Mₐ₂,** gegenüber dem Gehalt der organischen Verunreinigungen **V** in der Mischung **Mₐ₁,** bezogen auf den Gehalt aller Alkandiole der Formel **(I)** in der Mischung **Mₐ₁** verringert. Dies ist insbesondere dann der Fall, wenn Verunreinigungen **V** mit dem Zusatz **Z** reagieren und/oder adsorbiert werden. Werden sie adsorbiert, werden sie dann mit dem Zusatz **Z** abgetrennt.

Nach Durchführung des Unterschrittes (β2) wird in einer bevorzugten Ausführungsform eine Mischung **Mₐ₂** umfassend mindestens ein Alkandiol der Formel **(I)**, mindestens einen Zusatz **Z** und die organischen Verunreinigungen **V** erhalten, wobei der Gehalt des Zusatzes **Z** in der Mischung **Mₐ₂,** bezogen auf den Gehalt aller Alkandiole der Formel **(I)** in der Mischung **Mₐ₂,** gegenüber dem Gehalt des Zusatzes **Z** in der Mischung **Mₐ₁,** bezogen auf den Gehalt aller Alkandiole der Formel **(I)** in der Mischung **Mₐ₁,** um mindestens 50 %, bevorzugt um mindestens 75 %, bevorzugter um mindestens 90 % verringert ist.

Noch bevorzugter ist dann auch der Gehalt aller organischen Verunreinigungen **V** in der Mischung **Mₐ₂,** bezogen auf den Gehalt aller Alkandiole der Formel **(I)** in der Mischung **Mₐ₂,** gegenüber dem Gehalt aller organischen Verunreinigungen **V** in der Mischung **Mₐ₁,** bezogen auf den Gehalt aller Alkandiole der Formel **(I)** in der Mischung **Mₐ₁,** um mindestens 50 %, bevorzugt um mindestens 75 %, bevorzugter um mindestens 90 % verringert.

Es versteht sich von selbst, dass auch die Ausführungsform von der Erfindung umfasst ist, dass in Schritt (β2) der Zusatz **Z** nur teilweise aus der Mischung **Mₐ₁** abgetrennt wird. In einer weiteren bevorzugten Ausführungsform wird nach Durchführung des Unterschrittes (β2) eine Mischung **Mₐ₂** umfassend mindestens ein Alkandiol der Formel **(I)**, mindestens einen Zusatz **Z** und die organischen Verunreinigungen **V** erhalten, wobei der Gehalt des Zusatzes **Z** in der Mischung **Mₐ₂,** bezogen auf den Gehalt aller Alkandiole der Formel **(I)** in der Mischung **Mₐ₂,** gegenüber dem Gehalt des Zusatzes **Z** in der Mischung **Mₐ₁,** bezogen auf den Gehalt aller Alkandiole der Formel **(I)** in der Mischung **Mₐ₁,** um 50 % bis 99.99 %, bevorzugt um 75 % bis 99.0 %, bevorzugter um 90 % bis 98 % verringert ist.

Noch bevorzugter ist dann auch der Gehalt aller organischen Verunreinigungen **V** in der Mischung **Mₐ₂,** bezogen auf den Gehalt aller Alkandiole der Formel **(I)** in der Mischung **Mₐ₂,** gegenüber dem Gehalt aller organischen Verunreinigungen **V** in der Mischung **Mₐ₁,** bezogen auf den Gehalt aller Alkandiole der Formel **(I)** in der Mischung **Mₐ₁,** um 50 % bis 99.99 %, bevorzugt um 75 % bis 99.0 %, bevorzugter um 90 % bis 98 % verringert.

Aus der Mischung **M₃₂** wird dann im Unterschritt (β3) das mindestens eine Alkandiol der Formel **(I)** mindestens teilweise destillativ abgetrennt.

Die destillative Abtrennung des Alkandiols der Formel **(I)** kann nach dem Fachmann bekannten Verfahren erfolgen, wie zum Beispiel in der WO 2019/029808 A1, Absatz [0270] beschrieben.

Im Anschluss an die destillative Abtrennung wird dann die Zusammensetzung umfassend das gereinigte mindestens eine Alkandiol der Formel **(I)** erhalten.

### 2.1.3 Schritt (γ)

Als dritte Möglichkeit wird das Alkandiol der Formel **(I)** mindestens teilweise aus der Mischung **M** destillativ abgetrennt. Dabei wird während der destillative Abtrennung des Alkandiols der Formel **(I)** aus der Mischung **M** mindestens ein Zusatz **Z** zur Mischung **M** zugegeben.

Der Zusatz **Z** wird im Schritt (γ) dabei insbesondere in einer solchen Menge zugegeben, dass der Massenanteil aller Zusätze **Z** bezogen auf die Masse aller von der Mischung **M** umfassten Alkandiole **(I)** im Bereich 0.01 bis 10 Gew.-%, bevorzugt 0.1 bis 5 Gew.-%, bevorzugter 0.2 bis 3 Gew.-%, am bevorzugtesten 0.5 bis 2 Gew.-%, am allerbevorzugtesten 1 bis 1.5 Gew.-% liegt.

Die Zugabe des Zusatzes **Z** erfolgt in Schritt (γ) bevorzugt während der Destillation, bevor der Anteil aller im Destillationsrückstand verbliebenen Alkandiole der Formel **(I)** weniger als 50 %, bevorzugter weniger als 75 %, noch bevorzugter weniger als 90 % der Menge unterschreitet, die in der Mischung **M** eingesetzt wurden.

Es versteht sich von selbst, dass im erfindungsgemäßen Verfahren auch einer oder mehrere der Unterschritte kombiniert werden können. Beispielsweise kann der Schritt (γ) mit Schritt (a) so kombiniert werden, der Mischung **Mₐ₁** im Unterschritt (α2) gemäß Schritt (γ) Zusatz **Z** zugegeben wird. Alternativ kann der Schritt (γ) mit Schritt (β) so kombiniert werden, der Mischung **M₃₂** im Unterschritt (β3) gemäß Schritt (γ) Zusatz **Z** zugegeben wird.

### 2.2 Bevorzugtes Herstellungsverfahren der Mischung M

Die Mischung **M** wird bevorzugt wie folgt hergestellt:

### 2.2.1 Schritt (a) des bevorzugten erfindungsgemäßen Verfahrens

In Schritt (a) des bevorzugten erfindungsgemäßen Verfahrens wird ein Alken der Formel **(II)** 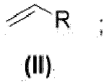 mit H₂O₂ und mindestens einer Säure **S** ausgewählt aus Ameisensäure, Essigsäure, bevorzugt Ameisensäure,
insbesondere in Gegenwart eines quartären Ammoniumsalzes der Formel **K_{N}A,** wobei **K_{N}** das Kation des quartären Ammoniumsalzes ist und **A** das Anion des quartären Ammoniumsalzes ist, umgesetzt.
R ist dabei ein unverzweigter oder verzweigter, bevorzugt unverzweigter Alkylrest mit 1 bis 10 Kohlenstoffatomen. Insbesondere ist R ein unverzweigter oder verzweigter, bevorzugt unverzweigter Alkylrest mit 1 bis 8 Kohlenstoffatomen. Bevorzugter ist R ein unverzweigter oder verzweigter, bevorzugt unverzweigter Alkylrest mit 1 bis 6 Kohlenstoffatomen. Noch bevorzugter ist R ein unverzweigter oder verzweigter, bevorzugt unverzweigter Alkylrest mit 1 bis 4 Kohlenstoffatomen. Noch mehr bevorzugter ist R ein unverzweigter oder verzweigter, bevorzugt unverzweigter Alkylrest mit 2 bis 3 Kohlenstoffatomen. Am bevorzugtesten ist R ein unverzweigter Kohlenstoffrest mit 3 Kohlenstoffatomen, dann handelt es sich bei der Struktur der Formel **(II)** um 1-Penten und bei der Struktur der Formel **(I)** um 1,2-*n*-Pentandiol.

H₂O₂ wird typischerweise als wässrige Lösung verwendet, wobei die Konzentration des H₂O₂ in der wässrigen Lösung üblicherweise bei 30 bis 50 Gew.-%, bevorzugt 50 Gew.-% liegt.

Insbesondere liegt das molare Verhältnis zwischen der Menge des in Schritt (a) des erfindungsgemäßen Verfahrens eingesetzten H₂O₂ zu der Menge aller in Schritt (a) des erfindungsgemäßen Verfahrens eingesetzten Verbindungen der Struktur **(I)** im Bereich 1 : 2 bis 3:1, bevorzugt 1 : 1 bis 2 : 1, am bevorzugtesten 1.25 : 1 bis 1.5 : 1.

In Schritt (a) wird außerdem mindestens eine Säure **S** ausgewählt aus Ameisensäure, Essigsäure eingesetzt. Bevorzugt wird Ameisensäure eingesetzt, da diese im Folgeschritt (b) besonders einfach durch Decarbonylierung entfernt werden kann.

Insbesondere liegt das molare Verhältnis zwischen der Gesamtmenge der in Schritt (a) des erfindungsgemäßen Verfahrens eingesetzten Säure **S** zu der Menge aller in Schritt (a) des erfindungsgemäßen Verfahrens eingesetzten Verbindungen der Struktur **(I)** im Bereich 1 : 1 bis 20 : 1, bevorzugt 1.5 : 1 bis 15 : 1, bevorzugter 2:1 bis 10 : 1, am bevorzugtesten 2 : 1 bis 2.5 : 1. Des Weiteren wird in einer bevorzugten Ausführungsform in Schritt (a) des erfindungsgemäßen Verfahrens ein quartäres Ammoniumsalz der Formel **K_{N}A,** wobei **K_{N}** das Kation des quartären Ammoniumsalzes ist und **A** das Anion des quartären Ammoniumsalzes ist, eingesetzt.

Der hier verwendete Begriff "quartäres Ammoniumkation **K_{N}"** soll sich auf ein Kation beziehen, das mindestens ein Stickstoffatom enthält, das eine positive elektrische Ladung trägt, wobei das Stickstoffatom nur an Kohlenstoffatome gebunden ist. Das Stickstoffatom kann gesättigt sein, also durch Einfachbindungen an vier Kohlenstoffatome gebunden sein, oder es kann ungesättigt sein, das heißt durch Einfachbindungen an zwei Kohlenstoffatome und durch eine Doppelbindung an ein drittes Kohlenstoffatom gebunden sein. Wenn das Stickstoffatom ungesättigt ist, kann es Teil eines heteroaromatischen Rings wie eines Imidazoliumkations oder Pyridiniumkations sein. Wenn das Stickstoffatom gesättigt ist, kann es Teil eines alicyclischen Rings sein, wie beispielsweise eines Pyrrolidinium- oder eines Piperidiniumkations. Vorzugsweise ist das Stickstoffatom an vier substituierte oder unsubstituierte Kohlenwasserstoffgruppen gebunden, die zusätzliche Substituenten an Kohlenstoffatomen tragen können, die nicht an das Stickstoffatom gebunden sind und eine positive elektrische Ladung tragen. Der Begriff "Kohlenwasserstoffgruppe" bezieht sich auf ein einwertiges Radikal, das von einem Kohlenwasserstoff abgeleitet ist und Alkyl-, Cycloalkyl-, Alkenyl-, Alkinyl- oder Arylgruppen einschließen kann.

Dabei ist **K_{N}** insbesondere ein Kation, welches aus der Gruppe bestehend aus den nachfolgenden Strukturen **(Q1), (Q2), (Q3), (Q4), (Q5),** mit 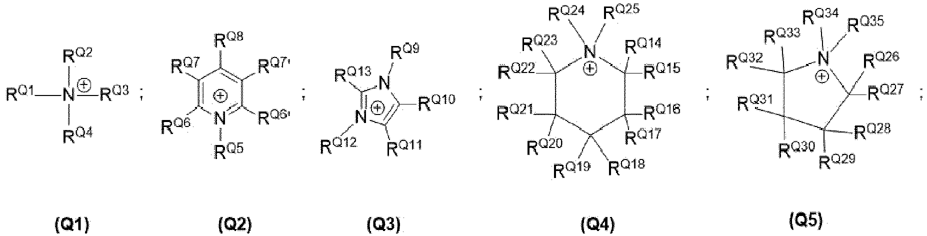 ausgewählt ist,
wobei R^{Q1}, R^{Q2}, R^{Q3}, R^{Q4}, unabhängig voneinander jeweils aus der Gruppe bestehend aus (Halo)Alkylgruppe, Cycloalkylgruppe ausgewählt sind,
und wobei R^{Q5}, R^{Q6}, R^{Q6'}, R^{Q7}, R^{Q7'}, R^{Q8}, R^{Q9}, R^{Q10}, R^{Q11}, R^{Q12}, R^{Q13}, R^{Q14}, R^{Q15}, R^{Q16}, R^{Q17}, R^{Q18}, R^{Q19}, R^{Q20}, R^{Q21}, R^{Q22}, R^{Q23}, R^{Q24}, R^{Q25}, R^{Q26}, R^{Q27}, R^{Q28}, R^{Q29}, R^{Q30}, R^{Q31}, R^{Q32}, R^{Q33}, R^{Q34}, R^{Q35} unabhängig voneinander jeweils aus der Gruppe bestehend aus Wasserstoff, (Halo)Alkylgruppe, die mindestens eine Ethergruppe aufweisen kann, Cycloalkylgruppe ausgewählt sind.
Bevorzugt ist **K_{N}** ein Kation, welches aus der Gruppe bestehend aus den Strukturen **(Q1), (Q2), (Q3), (Q4), (Q5)** ausgewählt ist, wobei R^{Q1}, R^{Q2}, R^{Q3}, R^{Q4} unabhängig voneinander jeweils aus der Gruppe bestehend aus Alkylgruppe mit 1 bis 40, bevorzugter 1 bis 30, noch bevorzugter 1 bis 18, noch bevorzugter 1 bis 10 Kohlenstoffatomen, Cycloalkylgruppe mit 6 bis 40, bevorzugter 10 bis 30 Kohlenstoffatomen ausgewählt sind,
wobei dann besonders bevorzugt R^{Q1} eine Alkylgruppe mit 1 bis 40 Kohlenstoffatomen, bevorzugter 1 bis 30 Kohlenstoffatomen, noch bevorzugter 1 bis 18 Kohlenstoffatomen, oder eine Cycloalkylgruppe mit 6 bis 40, bevorzugter 10 bis 30 Kohlenstoffatomen ist,
und R^{Q2}, R^{Q3}, R^{Q4} unabhängig voneinander jeweils aus der Gruppe bestehend aus Alkylgruppe mit 1 bis 40, bevorzugt 1 bis 35, bevorzugter 6 bis 35, noch bevorzugter 10 bis 30 Kohlenstoffatomen, noch bevorzugter 10 bis 18 Kohlestoffatomen, Cycloalkylgruppe mit 6 bis 40, bevorzugter 10 bis 30 Kohlenstoffatomen ausgewählt sind,
und wobei R^{Q5}, R^{Q6}, R^{Q6'}, R^{Q7}, R^{Q7'}, R^{Q8}, R^{Q9}, R^{Q10}, R^{Q11}, R^{Q12}, R^{Q13}, R^{Q14}, R^{Q15}, R^{Q16}, R^{Q17}, R^{Q18}, R^{Q19}, R^{Q20}, R^{Q21}, R^{Q22}, R^{Q23}, R^{Q24}, R^{Q25}, R^{Q26}, R^{Q27}, R^{Q28}, R^{Q29}, R^{Q30}, R^{Q31}, R^{Q32}, R^{Q33}, R^{Q34}, R^{Q35} unabhängig voneinander jeweils aus der Gruppe bestehend aus Wasserstoff, Alkylgruppe mit 1 bis 25, bevorzugt 1 bis 10 Kohlenstoffatomen, die mindestens eine Ethergruppe aufweisen kann, ausgewählt sind.
Bevorzugter ist **K_{N}** ein Kation, der Struktur **(Q1),** wobei R^{Q1}, R^{Q2}, R^{Q3}, R^{Q4}, unabhängig voneinander jeweils aus der Gruppe bestehend aus (Halo)Alkylgruppe, Cycloalkylgruppe ausgewählt sind, bevorzugt R^{Q1}, R^{Q2}, R^{Q3}, R^{Q4} unabhängig voneinander jeweils aus der Gruppe bestehend aus Alkylgruppe mit 1 bis 40, bevorzugter 1 bis 30, noch bevorzugter 1 bis 18 Kohlenstoffatomen, Cycloalkylgruppe mit 6 bis 40, bevorzugter 10 bis 30 Kohlenstoffatomen ausgewählt sind,
wobei dann besonders bevorzugt R^{Q1} eine Alkylgruppe mit 1 bis 40 Kohlenstoffatomen, bevorzugter 1 bis 30 Kohlenstoffatomen, noch bevorzugter 1 bis 18 Kohlenstoffatomen, oder eine Cycloalkylgruppe mit 6 bis 40, bevorzugter 10 bis 30 Kohlenstoffatomen ist,
und R^{Q2}, R^{Q3}, R^{Q4} unabhängig voneinander jeweils aus der Gruppe bestehend aus Alkylgruppe mit 1 bis 40, bevorzugt 1 bis 35, bevorzugter 1 bis 30, noch bevorzugter 1 bis 25 Kohlenstoffatomen, noch bevorzugter 1 bis 18 Kohlenstoffatomen, Cycloalkylgruppe mit 6 bis 40, bevorzugter 10 bis 30 Kohlenstoffatomen ausgewählt sind.

Weitere bevorzugte Kationen **K_{N}** sind solche der Struktur **(Q1),** in denen R^{Q1} eine Alkylgruppe mit 1 bis 40 Kohlenstoffatomen, bevorzugter 1 bis 30 Kohlenstoffatomen, noch bevorzugter 1 bis 18 Kohlenstoffatomen, noch mehr bevorzugt mit 1 bis 4 Kohlenstoffatomen und noch viel mehr bevorzugter Methyl oder Butyl und am allerbevorzugtesten Methyl ist,
und R^{Q2}, R^{Q3}, R^{Q4} unabhängig voneinander jeweils aus der Gruppe bestehend aus Alkylgruppe mit 1 bis 40, bevorzugt 1 bis 35, bevorzugter 1 bis 30, noch bevorzugter 1 bis 25 Kohlenstoffatomen, noch bevorzugter 1 bis 18 Kohlenstoffatomen, noch mehr bevorzugter 4 bis 18 und am bevorzugtesten 4 bis 16 Kohlenstoffatomen ausgewählt sind.

Am bevorzugtesten ist Tetrabutylammonium als Kation **K_{N}**.

Der hier verwendete Begriff "Anion **A**" soll sich auf das Anion des quartären Ammoniumsalzes beziehen, welches insbesondere aus der Gruppe bestehend aus Phosphat, Phosphonat, Alkylphosphonat, Monoalkylphosphat, Dialkylphosphat, Bis[trifluormethansulfonyl]imid, (Halo)alkylsulfonat, (Halo)alkylsulfat, Bis[fluorsulfonyl]imid, Halogenid, Dicyanamid, Hexafluorophosphat, Sulfat, Tetrafluoroborat, Trifluormethansulfonat, Perchlorat, Hydrogensulfat, (Halo)alkylcarboxylat, Bisoxalatoborat, Tetrachloroaluminat, Dihydrogenphosphat, Monoalkylhydrogenphosphat, Nitrat, Monocarboxylat, Hydroxid, Alkoxid, Alkylcarbonat, Hydrogencarbonat, Carbonat, Serinat, Prolinat, Histidinat, Threoninat, Valinat, Asparaginat, Taurinat, Lysinat ausgewählt ist,
bevorzugt aus der Gruppe bestehend aus Methylsulfat, Halogenid, Monocarboxylat, Hydrogensulfat, Hydroxid, Alkoxid, Alkylcarbonat, Hydrogencarbonat, Carbonat ausgewählt ist, bevorzugter aus der Gruppe bestehend aus Methylsulfat, Chlorid, Bromid, Jodid, Formiat, Acetat ausgewählt ist,
noch bevorzugter aus der Gruppe bestehend aus Chlorid, Bromid, Formiat, Methylsulfat, Acetat, Hydrogensulfat ausgewählt ist,
noch bevorzugter aus der Gruppe bestehend aus Formiat, Acetat, Bromid, Chlorid, Hydrogensulfat ausgewählt ist,
noch bevorzugter aus der Gruppe bestehend aus Formiat, Acetat, Bromid, Chlorid ausgewählt ist,
noch mehr bevorzugter aus der Gruppe bestehend aus Formiat, Acetat ausgewählt ist.
Am bevorzugtesten ist "Anion **A**" dann Formiat, wenn in Schritt (a) des erfindungsgemäßen Verfahrens Ameisensäure als Säure **S** eingesetzt wird,
und "Anion **A**" dann Acetat eingesetzt, wenn in Schritt (a) des erfindungsgemäßen Verfahrens Essigsäure als Säure **S** eingesetzt wird.

"Monocarboxylat" bedeutet erfindungsgemäß das zu einer Monocarbonsäure korrespondierende Anion, und ist bevorzugt ausgewählt aus der Gruppe bestehend aus Formiat, Acetat.

Insbesondere liegt das molare Verhältnis zwischen der Gesamtmenge der in Schritt (a) des erfindungsgemäßen Verfahrens eingesetzten quartären Ammoniumsalze der Formel **K_{N}A** zu der Menge aller in Schritt (a) des erfindungsgemäßen Verfahrens eingesetzten Verbindungen der Struktur **(II)** im Bereich 0.0001 : 1 bis 1 : 1, bevorzugt 0.001 : 1 bis 0.1 : 1, am bevorzugtesten 0.025 : 1.

"Halogenid" ist ausgewählt aus Fluorid, Bromid, Chlorid, Jodid, bevorzugter ausgewählt aus Chlorid, Bromid, am bevorzugtesten Chlorid.

In Schritt (a) des bevorzugten erfindungsgemäßen Verfahrens wird ein Rohprodukt **RP₁** erhalten wird, welches das quartäre Ammoniumsalz **K_{N}A** und mindestens eine Verbindung ausgewählt aus der Gruppe bestehend aus Monoformiate **(III-A)** und **(III-B),** Diformiat **(III-C)** mit 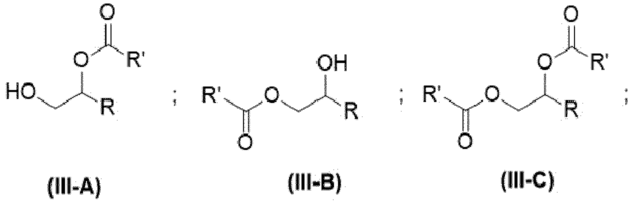 umfasst, wobei R' = Methyl oder Wasserstoff. R' ist Methyl, wenn in Schritt (a) des erfindungsgemäßen Verfahrens als Säure **S** Essigsäure eingesetzt wurde. R' ist Wasserstoff, wenn in Schritt (a) des erfindungsgemäßen Verfahrens als Säure **S** Ameisensäure eingesetzt wurde.

Da der Einsatz von Ameisensäure als Säure **S** in Schritt (a) des erfindungsgemäßen Verfahrens bevorzugt ist, ist auch R' = Wasserstoff bevorzugt.

Schritt (a) wird ansonsten nach dem Fachmann bekannten Methoden durchgeführt. Die Reaktion vom Alken der Formel **(II)** zu den Monoformiaten **(III-A)** und **(III-B)** bzw. dem Diformiat **(III-C)** verläuft über das Epoxid, wie in der DE 199 29 196 A1 beschrieben.

Die Apparatur für die Umsetzung in Schritt (a) ist dem Fachmann bekannt. So kann diese in einer Standardapparatur durchgeführt werden (Reaktionskolben mit Rückflusskühler oder für großtechnische Anwendungen Reaktoren).

Die Umsetzung nach Schritt (a) erfolgt bevorzugt bei einer Temperatur von 50 °C bis 100 °C, bevorzugter 60 °C bis 70 °C und einem Druck von 0.5 bis 1.5 bar, bevorzugt 1 bar.

Schritt (a) läuft insbesondere in wässriger Lösung ab.

Gegebenenfalls werden in Schritt (a) auch organische Lösungsmittel wie insbesondere Toluol, Xylol, Cumol oder Benzol zugegeben.

Die Reaktionsdauer hängt von der Geschwindigkeit des Umsatzes ab und ist bevorzugt zwischen 4 bis 10 Stunden, bevorzugter 5 bis 6 Stunden. Bevorzugt wird die Reaktion durchgeführt, bis sich mindestens 50 Mol.-%, bevorzugter mindestens 70 Mol.-%, noch bevorzugter mindestens 80 Mol.-%, noch mehr bevorzugter mindestens 90 Mol.-%, noch viel mehr bevorzugter mindestens 90 Mol.-%, am bevorzugtesten mindestens 99 Mol.-% der in Schritt (a) eingesetzten Verbindung der Struktur **(II)** zu den nachfolgend genannten Verbindungen **(III-A), (III-B)** bzw. **(III-C)** umgesetzt hat.

Nach Beendigung der Reaktion umfasst das Rohprodukt **RP₁** noch Wasser und gegebenenfalls organisches Lösungsmittel. Daneben umfasst es auch gegebenenfalls noch Ameisensäure und/oder Essigsäure. Es ist vorteilhaft, diese Bestandteile vor Durchführung des Schrittes (b) aus dem Rohprodukt **RP₁** mindestens teilweise zu entfernen. Dies erfolgt insbesondere destillativ. Bevorzugt kann dazu, wie in der DE 199 29 196 A1 beschrieben, ein organisches Lösungsmittel vor oder kontinuierlich während der Destillation zugefügt werden, um überschüssiges Wasser, Ameisensäure, Essigsäure zu entfernen.

Bei der destillativen Entfernung wird bevorzugt mindestens 50 Mol.-%, bevorzugter mindestens 70 Mol.-%, noch bevorzugter mindestens 80 Mol.-%, noch mehr bevorzugter mindestens 90 Mol.-%, noch viel mehr bevorzugter mindestens 90 Mol.-%, am bevorzugtesten mindestens 99 Mol.-% des Wassers, was **RP₁** direkt nach Beendigung des Schrittes (a) umfasst, entfernt.

Bei der destillativen Entfernung wird bevorzugt mindestens 50 Mol.-%, bevorzugter mindestens 70 Mol.-%, noch bevorzugter mindestens 80 Mol.-%, noch mehr bevorzugter mindestens 90 Mol.-%, noch viel mehr bevorzugter mindestens 90 Mol.-%, am bevorzugtesten mindestens 99 Mol.-% der Ameisensäure, die **RP₁** direkt nach Beendigung des Schrittes (a) umfasst, entfernt.

Bei der destillativen Entfernung wird bevorzugt mindestens 50 Mol.-%, bevorzugter mindestens 70 Mol.-%, noch bevorzugter mindestens 80 Mol.-%, noch mehr bevorzugter mindestens 90 Mol.-%, noch viel mehr bevorzugter mindestens 90 Mol.-%, am bevorzugtesten mindestens 99 Mol.-% der Essigsäure, die **RP₁** direkt nach Beendigung des Schrittes (a) umfasst, entfernt.

### 2.2.2 Schritt (b) des erfindungsgemäßen Verfahrens

In Schritt (b) des bevorzugten erfindungsgemäßen Verfahrens wird die mindestens eine Verbindung ausgewählt aus der Gruppe bestehend aus Monoester **(III-A)** und **(III-B),** Diester **(III-C)** im Rohprodukt **RP₁** verseift.

"Verseifung" bedeutet erfindungsgemäß die basische Spaltung der Verbindungen **(III-A), (III-B)** bzw. **(III-C)** zum 1,2-Alkandiol der Formel **(I)**. "Verseifung" schließt erfindungsgemäß auch den Begriff der "Decarbonylierung" ein.

Der Schritt (b) erfolgt nach dem Fachmann bekannten Methoden. Insbesondere wird ein Verseifungsreagenz, bevorzugt Alkalicarbonat, Alkalihydroxid, Alkalialkoholat, bevorzugt Alkalialkoholat zugegeben und dieses bei 10 °C bis 100 °C, bevorzugt 20 °C bis 70 °C mit dem Rohprodukt reagieren gelassen.

Bevorzugtes Alkalialkoholat ist dabei Alkalimethanolat, noch bevorzugter Natriummethanolat, noch mehr bevorzugter eine methanolische Lösung von Natriummethanolat.
Bevorzugtes Alkalihydroxid ist Natriumhydroxid, insbesondere eine wässrige Lösung von Natriumhydroxid.
Bevorzugtes Alkalicarbonat ist Natriumcarbonat, insbesondere eine wässrige Lösung von Natriumcarbonat.

Insbesondere wird dabei so lange Verseifungsreagenz zugegeben, bis sich ein pH von 7.1 bis 14, bevorzugt 8 bis 12, bevorzugter 10 bis 11 einstellt. Die Verseifungsreaktion ist üblicherweise nach 5 Minuten bis 10 Stunden, bevorzugter 0.5 bis 2 Stunden beendet.

Falls in Schritt (a) als Säure **S** Ameisensäure eingesetzt wurde, handelt es sich bei den Verbindungen **(III-A), (III-B)** um Monoformiate und bei der Verbindung **(III-C)** um das Diformiat.

In diesem Fall ist es außerdem bevorzugt, eine Decarbonylierung durchzuführen, wie sie beispielsweise in der WO 2019/029808 A1, Absätze [0015], [0016] beschrieben ist.

Besonders bevorzugt ist es dann, eine Decarbonylierung wie in DE 32 29 084 A1 durchzuführen. Demnach wird dem Rohprodukt **RP₁** umfassend mindestens einen Verbindung ausgewählt aus den Monoformiaten **(III-A), (III-B)** und dem Diformiat **(III-C)** mit einem basischen Katalysator **Kat_{B}** ausgewählt aus Alkali- oder Erdalkalialkoholaten von Alkoholen mit 1 bis 10, bevorzugt 1 bis 4 Kohlenstoffatomen versetzt. Sehr bevorzugt sind dabei Methylate, Ethylate, *tert*-Butylate von Natrium, Kalium, gegebenenfalls Calcium. Besonders bevorzugt sind Methylate des Natriums, Kaliums, besonders bevorzugt Natriummethanolat.

Die Menge von in Schrit (b) eingesetztem **Kat_{B}** bezogen auf die Menge der in Schritt (a) eingesetzten Verbindung der Formel **(II)** beträgt dann insbesondere 0.1 bis 15 Mol-%, vorzugsweise 0.5 bis 13.5 Mol.-%, noch bevorzugter 1 bis 10 Mol.-%.

Die Decarbonylierung mit **Kat_{B}** wird typischerweise durch Zugabe von **Kat_{B}** zum Rohprodukt **RP₁** und insbesondere Erhitzen auf eine Temperatur von 80 °C bis 200 °C, bevorzugt 100 °C bis 170 °C durchgeführt.

Dabei entsteht Kohlenmonoxid, anhand dessen der Fortgang und die Beendigung der Reaktion kontrolliert werden kann. Bevorzugt wird die Decarbonylierung durchgeführt, bis kein CO mehr entsteht.

Am Ende des Schrittes (b) wird ein zweites Rohprodukt **RP₂** umfassend das Ammoniumsalz **K_{N}A** und das 1,2-Alkandiol **(I)** erhalten.

### 2.2.3 Schritt (c) des bevorzugten erfindungsgemäßen Verfahrens

In Schritt (c) des bevorzugten erfindungsgemäßen Verfahrens wird das 1,2-Alkandiol **(I)** mindestens teilweise aus **RP₂** destillativ abgetrennt.

Die destillative Abtrennung kann nach dem Fachmann bekannten Methoden erfolgen. Die eingestellte Temperatur und der eingestellte Druck, bei dem die Destillation erfolgt, ist abhängig von der jeweiligen Struktur des abzutrennenden 1,2-Alkandiols **(I)** und kann vom Fachmann eingestellt werden.

Am Ende des Schrittes (c) erhält man dann die Mischung **M** umfassend das 1,2-Alkandiol **(I)**. Die Mischung **M** umfasst daneben auch die organischen Verunreinigungen **V**, die sich während Schritt (a) und/oder Schritt (b) und/oder (c) bilden und in Schritt (c) mindestens teilweise mit dem 1,2-Alkandiols **(I)** aus **RP₂** abgetrennt werden.

Wenn Schritt (a) in Gegenwart eines quartären Ammoniumsalzes der Formel **K_{N}A** durchgeführt wird, umfassen die organischen Verunreinigungen **V** insbesondere auch aminische organische Verunreinigungen **V.**

### 2.3 Zusatz Z

Der Zusatz **Z** umfasst mindestens eine polymere Sulfonsäure.

Dies sind insbesondere anorganische, beispielsweise Siliciumdioxid, oder organische Polymere, beispielsweise Polystyrol oder Polystyrolcopolymere, wie Styrol-Divinylbenzol-Copolymere, die protische Sulfonsäuregruppen, insbesondere Alkylsulfonsäureestergruppen oder Sulfonsäureestergruppen (-SO₃⁻), insbesondere Sulfonsäureestergruppen aufweisen.

Dies sind bevorzugt organische Polymere, beispielsweise Polystyrol oder Polystyrolcopolymere, wie Styrol-Divinylbenzol-Copolymere, die protische Sulfonsäuregruppen, insbesondere Alkylsulfonsäureestergruppen, oder Sulfonsäureestergruppen (-SO₃⁻), insbesondere Sulfonsäureestergruppen, aufweisen.

"Protische Sulfonsäuregruppe" bedeutet dabei, dass die Gruppe eine freie saure "-OH" Funktion aufweist, die ein Proton abspalten kann. Das Wort "Ester" in "Sulfonsäure**ester**gruppe" bzw. "Alkylsulfonsäure**ester**gruppen" wird in diesem Zusammenhang deshalb benutzt, um zu definieren, dass das Schwefelatom direkt (Sulfonsäure**ester**gruppe) oder über eine Alkylgruppe (Alkylsulfonsäure**ester**gruppe) an das Polymer gebunden ist.

Ein bevorzugter Zusatz **Z** ist ausgewählt aus der folgenden Gruppe:
- Ein Katalysator, der ein Polymerharz, insbesondere Polystyrol oder Styrol-Divinylbenzol-Copolymer, bevorzugt Polystyrol, und -SO₃⁻-Gruppen als funktionelle Gruppen aufweist (käuflich erwerblich als "Amberlyst® 15", "Amberlite® 200", "Lewatit® SP 120" bzw. "Lewatit® K2621"); daneben ist auch Polystyrolsulfonat mit der CAS-Nummer: 28210-41-5 einsetzbar;
- Ein Katalysator, der Sulfonsäure in polymerer Form aufweist und perfluoriert sein kann (beschrieben in DE 10 2010 062 804 A1, US 4,831,146). Dieser kann zum Beispiel ein sulfoniertes Tetrafluorethylen (CAS-Nummer: 31175-20-9) oder eine feste geträgerte perfluorierte Sulfonsäure in polymerer Form mit Siliciumdioxid als Trägermaterial sein. Derartige Katalysatoren sind unter anderem unter den Handelsnamen Nafion®, Aciplex® F, Femion®, Neosepta®, Fumion® F erhältlich. Ein bevorzugter Katalysator ist Nafion® SAC-13. Bei Nafion® SAC-13 handelt es sich um poröse Siliciumdioxidpartikel, auf dem Nafion® in einer Beladung von etwa 13 Gew.-% adsorbiert worden ist;
- Poly(2-acrylamido-2-methyl-1-propanesulfonsäure), vertrieben als PolyAMPS® von Lubrizol.

Ein bevorzugter Zusatz **Z** ist Polystyrol oder Styrol-Divinylbenzol-Copolymer, bevorzugter Styrol-Divinylbenzol-Copolymer, welches -SO₃⁻-Gruppen als funktionelle Gruppen aufweist.

Nach Durchführung der erfindungsgemäßen Desodorierung erhält man dann ein reines 1,2-Alkandiol, das heißt in dem Endprodukt liegt der Massenanteil des 1,2-Alkandiols nach Formel **(I)** bei mindestens 80 Gew.-%, bevorzugt mindestens 90 Gew.-%.

Dieses Endprodukt eignet sich zur Anwendung in Kosmetika oder Haushaltsreinigern und anderen Haushaltsartikeln. Weitere Anwendungsgebiete sind in der WO 2019/029808 A1 aufgezählt.

Die nachfolgenden Beispiele sollen die vorliegende Erfindung illustrieren, ohne sie zu beschränken.

### 3. Beispiele

### 3.1 Herstellung des Rohproduktes

Das Rohprodukt wird in Anlehnung an Lewandowski und DE 32 29 084 A1 hergestellt.

Es werden sieben Ansätze gefahren. Jeweils werden zu einer Mischung von 25 kg 1-Penten (350 mol; CAS-Nummer: 109-67-1) und 200 I (244 kg, 5300 mol, 15 molare Äquivalente) Ameisensäure, 50 kg einer 30-Gew.-%igen wässrigen H₂O₂-Lösung (entspricht 440 mol H₂O₂, 1.25 molare Äquivalente bezogen auf 1-Penten) zugegeben. Zu jedem Ansatz gibt man das folgende quartäre Ammoniumsalz in einer Menge von 8.75 mol.
**Ansatz 1:** Methyltrioctylammoniumchlorid CH₃(C₈H₁₇)₃NCl (CAS-Nummer 5137-55-3).
**Ansatz 2:** Methyltrioctylammoniumbromid CH₃(C₈H₁₇)₃NBr (CAS-Nummer: 35675-80-0).
**Ansatz 3:** Cetylpyridiniumchlorid (CAS-Nummer: 6004-24-6).
**Ansatz 4:** Cetylpyridiniumbromid (CAS-Nummer: 202869-92-9).
**Ansatz 5**: Tetrabutylammoniumhydrogensulfat (CAS-Nummer: 32503-27-8).
**Ansatz 6**: Dimethyl[dioctadecyl(76%) +dihexadecyl(24%)] ammoniumchlorid (Arquad® 2HT-75; CAS-Nummer: 61789-80-8).
**Ansatz 7**: Didodecyldimethylammoniumbromid (CAS-Nummer: 3282-73-3).

Jeder Ansatz wird bis zur vollständigen Umsetzung des 1-Pentens umgesetzt. Dann werden Wasser und überschüssige Ameisensäure destillativ entfernt. Jedem Rückstand werden 3 kg Kalium-*tert*-Butylat (26 mol, 13.5 Mol-% bezogen auf eingesetztem 1-Penten;
CAS-Nummer: 865-47-4) zugefügt und bei 150 °C bis 160 °C gerührt, bis keine Gasbildung mehr beobachtet werden kann.

Aus jedem Ansatz wird dann 1,2-Pentandiol abdestilliert. Das Destillat wird aufgeteilt und jeweils 500 g des Destillats mit 5 g der folgenden Zusätze (**E1, E2:** 0.5 g) versetzt:

**Tabelle für Anmeldung 3:**

| **Versuch** | **Zugabe** |
|---|---|
| **V1** | ohne |
| **V2** | ohne |
| **V3** | Silica Gel |
| **V4** | Aktivkohle, gekörnt |
| **E1** | Amberlyst |
| **E2** | Amberlyst |

Amberlyst ist ein Styrol-Divinylbenzol-Copolymer, welches mit -SO₃⁻-Gruppen funktionalisiert ist.

Danach wird bei den Beispielen **V1, V2, V3, V4** aus der erhaltenen Mischung wiederum 1,2-Alkandiol destilliert, das Destillat über Nacht stehen gelassen und danach der Geruch kontrolliert.

Im Beispiel **E1, E2** wurde der jeweilige Zusatz der Probe zugegeben, die erhaltene Mischung 60 Minuten gerührt und die zu destillierende Lösung vor der Destillation abdekantiert.

Die olfaktorische Beurteilung erfolgte wie folgt:
Es wurden 45.0 g VE-Wasser in einem Weithals-Erlenmeyerkolben vorgelegt, im Trockenschrank auf 60 °C Wassertemperatur erwärmt (Umlufttrockenschrank 63 °C) und 5.0 g der jeweiligen Probe hinzugefügt. Der Geruch wurden sodann beurteilt.

Für jeden Ansatz wurden je nach Zusatz die gleichen geruchlichen Ergebnisse festgestellt. Diese waren wie folgt:

| **Versuch** | **Zugabe** | **Ergebnis** |
|---|---|---|
| **V1** | ohne | schlecht, A |
| **V2** | ohne | schlecht, A |
| **V3** | Silica Gel | schlecht, wenig A |
| **V4** | Aktivkohle, gekörnt | A |
| **E1** | Amberlyst | gut, wenig A |
| **E2** | Amberlyst | gut |

Während bei den Vergleichsbeispielen **V1** bis **V4** ein schlechter Geruch des 1,2-Pentandiols beobachtet wurde, der sich insbesondere in einem stark aminischen Geruch äußerte (Abkürzung "A"), waren die gemäß den erfinderischen Beispielen **E1** und **E2** behandelten Proben der Ansätze 1 bis 7 geruchsfrei bzw. der üble Geruch deutlich reduziert. Dies war vollkommen überraschend.

## Patentansprüche

1. Verfahren zur Desodorierung mindestens eines Alkandiols der Formel **(I)** mit 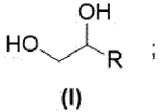 wobei R ein unverzweigter oder verzweigter Alkylrest mit 1 bis 10 Kohlenstoffatomen ist,
wobei das mindestens eine Alkandiol der Formel **(I)** in einer Mischung **M** umfassend mindestens ein Alkandiol der Formel **(I)** und organische Verunreinigungen **V** bereitgestellt wird, und gemäß dem Schritt (α), (β) oder (γ) mindestens teilweise von den organische Verunreinigungen **V** abgetrennt wird:
(α) (α1) Vermischen der Mischung **M** mit mindestens einem Zusatz **Z,** wodurch eine Mischung **Mₐ₁** umfassend mindestens ein Alkandiol der Formel **(I)**, mindestens einen Zusatz **Z** und die organischen Verunreinigungen **V** erhalten wird,
(α2) und mindestens teilweise destillative Abtrennung des mindestens einen Alkandiols der Formel **(I)** aus der Mischung **Mₐ₁;**
(β) (β1) Vermischen der Mischung **M** mit mindestens einem Zusatz **Z,** wodurch eine Mischung **Mₐ₁** umfassend mindestens ein Alkandiol der Formel **(I)**, mindestens einen Zusatz **Z** und die organischen Verunreinigungen **V** erhalten wird,
(β2) mindestens teilweise Abtrennung des Zusatzes **Z** aus der Mischung **Mₐ₁,** wodurch eine Mischung **Mₐ₂** umfassend mindestens ein Alkandiol der Formel **(I)**, mindestens einen Zusatz **Z** und die organischen Verunreinigungen **V** erhalten wird, wobei der Gehalt des Zusatzes **Z** in der Mischung **Mₐ₂** gegenüber **Mₐ₁** verringert ist,
(β3) und mindestens teilweise destillative Abtrennung des mindestens einen Alkandiols der Formel **(I)** aus der Mischung **Mₐ₂;**
(γ) mindestens teilweise destillative Abtrennung des mindestens einen Alkandiols der Formel **(I)** aus der Mischung **M,** wobei während der destillative Abtrennung des Alkandiols der Formel **(I)** aus der Mischung **M** mindestens ein Zusatz **Z** zur Mischung **M** zugegeben wird;
**dadurch gekennzeichnet, dass** der Zusatz **Z** mindestens eine polymere Sulfonsäure umfasst.

2. Verfahren nach Anspruch 1, in der der Zusatz **Z** im Unterschritt (α1) oder im Unterschritt (β1) oder im Schritt (γ) in einer solchen Menge zugegeben wird, dass der Massenanteil aller Zusätze **Z** bezogen auf die Masse aller von der Mischung **M** umfassten Alkandiole **(I)** im Bereich 0.01 bis 10 Gew.-% liegt.

3. Verfahren nach Anspruch 1 oder 2, wobei die organischen Verunreinigungen **V** aus der Gruppe bestehend aus Lactonen, Aminen, Ketonen ausgewählt sind.

4. Verfahren nach einem der Ansprüche 1 bis 3, wobei die Mischung **M** wie folgt erhalten wird:
(a) Umsetzung eines Alkens der Formel **(II)** 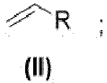 mit H₂O₂ und mindestens einer Säure **S** ausgewählt aus Ameisensäure, Essigsäure,
insbesondere in Gegenwart eines quartären Ammoniumsalzes der Formel **K_{N}A,**
wobei **K_{N}** das Kation und **A** das Anion des quartären Ammoniumsalzes ist,
wodurch ein Rohprodukt **RP₁** erhalten wird, welches mindestens eine Verbindung ausgewählt aus der Gruppe bestehend aus Monoformiate **(III-A)** und **(III-B),** Diformiat **(III-C)** mit 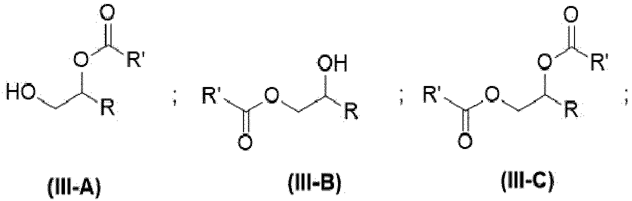 umfasst, wobei R' = Methyl oder Wasserstoff,
und wobei das Rohprodukt **RP₁** gegebenenfalls das quartäre Ammoniumsalz **K_{N}A** umfasst,
(b) Verseifung der mindestens einen Verbindung ausgewählt aus der Gruppe bestehend aus Monoester **(III-A)** und **(III-B),** Diester **(III-C)** im Rohprodukt **RP₁,**
wodurch ein zweites Rohprodukt **RP₂** umfassend das 1,2-Alkandiol **(I)** und gegebenenfalls das quartäre Ammoniumsalz **K_{N}A** erhalten wird,
(c) mindestens teilweise, bevorzugt destillative, Abtrennung des 1,2-Alkandiols **(I)** aus **RP₂,**
wobei sich organische Verunreinigungen **V** während Schritt (a) und/oder Schritt (b) und/oder (c) bilden und in Schritt (c) mindestens teilweise mit dem 1,2-Alkandiols **(I)** aus **RP₂** abgetrennt werden,
wodurch im Anschluss an Schritt (c) die Mischung **M** erhalten wird.

5. Verfahren nach Anspruch 4, wobei Schritt (a) in Gegenwart eines quartären Ammoniumsalzes der Formel **K_{N}A,** wobei **K_{N}** das Kation und **A** das Anion des quartären Ammoniumsalzes ist, durchgeführt wird.

6. Verfahren nach Anspruch 5, wobei **K_{N}** ein Kation ist, welches aus der Gruppe bestehend aus den nachfolgenden Strukturen **(Q1), (Q2), (Q3), (Q4), (Q5),** mit 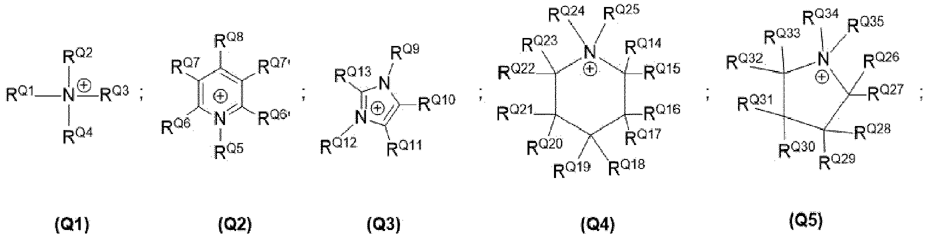 ausgewählt ist,
wobei R^{Q1}, R^{Q2}, R^{Q3}, R^{Q4}, unabhängig voneinander jeweils aus der Gruppe bestehend aus (Halo)Alkylgruppe, Cycloalkylgruppe ausgewählt sind,
und wobei R^{Q5}, R^{Q6}, R^{Q6'}, R^{Q7}, R^{Q7'}, R^{Q8}, R^{Q9}, R^{Q10}, R^{Q11}, R^{Q12}, R^{Q13}, R^{Q14}, R^{Q15}, R^{Q16}, R^{Q17}, R^{Q18}, R^{Q19}, R^{Q20}, R^{Q21}, R^{Q22}, R^{Q23}, R^{Q24}, R^{Q25}, R^{Q26}, R^{Q27}, R^{Q28}, R^{Q29}, R^{Q30}, R^{Q31}, R^{Q32}, R^{Q33}, R^{Q34}, R^{Q35} unabhängig voneinander jeweils aus der Gruppe bestehend aus Wasserstoff, (Halo)Alkylgruppe, die mindestens eine Ethergruppe aufweisen kann, Cycloalkylgruppe ausgewählt sind.

7. Verfahren nach Anspruch 5 oder 6, wobei Anion **A** aus der Gruppe bestehend aus Phosphat, Phosphonat, Alkylphosphonat, Monoalkylphosphat, Dialkylphosphat, Bis[trifluormethansulfonyl]imid, Haloalkylsulfonat, Alkylsulfonat, Alkylsulfat, Haloalkylsulfat, Bis[fluorsulfonyl]imid, Halogenid, Dicyanamid, Hexafluorophosphat, Sulfat, Tetrafluoroborat, Trifluormethansulfonat, Perchlorat, Hydrogensulfat, Alkylcarboxylat, Haloalkylcarboxylat, Bisoxalatoborat, Tetrachloroaluminat, Dihydrogenphosphat, Monoalkylhydrogenphosphat, Nitrat, Monocarboxylat, Hydroxid, Alkoxid, Alkylcarbonat, Hydrogencarbonat, Carbonat, Serinat, Prolinat, Histidinat, Threoninat, Valinat, Asparaginat, Taurinat, Lysinat ausgewählt ist.

8. Verfahren nach einem der Ansprüche 5 bis 7, wobei das molare Verhältnis zwischen der Gesamtmenge der in Schritt (a) eingesetzten quartären Ammoniumsalze der Formel **K_{N}A** zu der Menge aller in Schritt (a) eingesetzten Verbindungen der Struktur **(II)** im Bereich 0.0001 : 1 bis 1 : 1 liegt.

9. Verfahren nach einem der Ansprüche 1 bis 8, wobei R ein unverzweigter oder verzweigter Alkylrest mit 1 bis 8 Kohlenstoffatomen ist.

10. Verfahren nach einem der Ansprüche 1 bis 9, wobei der Zusatz **Z** mindestens ein organisches Polymer ist, das protische Sulfonsäuregruppen aufweist.
